**Europäisches Patentamt**

**European Patent Office**

**Office européen des brevets**

(19)

(11) Veröffentlichungsnummer : **0 334 235 B1**

(12)

# EUROPÄISCHE PATENTSCHRIFT

(45) Veröffentlichungstag der Patentschrift :
**26.08.92 Patentblatt 92/35**

(51) Int. Cl.$^5$ : **G01L 19/00, A61L 2/24**

(21) Anmeldenummer : **89104827.4**

(22) Anmeldetag : **17.03.89**

(54) **Druckmesseinrichtung.**

(30) Priorität : **25.03.88 DE 8804105 U**

(43) Veröffentlichungstag der Anmeldung :
**27.09.89 Patentblatt 89/39**

(45) Bekanntmachung des Hinweises auf die
Patenterteilung :
**26.08.92 Patentblatt 92/35**

(84) Benannte Vertragsstaaten :
**AT BE CH DE FR IT LI LU NL**

(56) Entgegenhaltungen :
**DE-A- 2 108 089**
**GB-A- 760 538**
**US-A- 1 640 600**

(73) Patentinhaber : **M M M MUENCHENER**
**MEDIZIN MECHANIK GMBH**
**Halmstrasse 6**
**W-8000 München 70 (DE)**

(72) Erfinder : **Büchner, Robert**
**Kirchseeoner Weg 39**
**W-8031 Eglharting (DE)**
Erfinder : **Kammermeier, Bernhard**
**Thalkirchner Strasse 16**
**W-8000 München 70 (DE)**

(74) Vertreter : **Patentanwälte Grünecker,**
**Kinkeldey, Stockmair & Partner**
**Maximilianstrasse 58**
**W-8000 München 22 (DE)**

EP 0 334 235 B1

## Beschreibung

Die Erfindung bezieht sich auf eine Druckmeßeinrichtung der im Oberbegriff des Patentanpruchs 1 erläuterten Art.

Eine derartige Druckmeßeinrichtung ist aus der GB-A-760 538 bekannt. Bei der bekannten Druckmeßvorrichtung soll der Einfluß der Höhe einer Kondensatsäule auf das Meßergebnis eliminiert und ein Meßinstrument gegen hohe Temperaturen geschützt werden. Zu diesem Zweck ist eine V-förmig gebogene, vertikal ausgerichtete Druckleitung zwischen dem Druckbehälter und dem Druckfühler vorgesehen, an deren nach unten weisender Umlenkstelle über eine Verbindungsleitung ein Kondensattopf genügender Tiefe vorgesehen ist, der gegenüber der Atmosphäre offen ist, wobei jedoch über die Länge der Verbindungsleitung und die Tiefe ihres Eindringens in den Kondensattopf die Druckleitung gegenüber der Atmosphäre abgedichtet werden soll. Das Kondensatniveau muß jedoch für eine ordnungsgemäße Funktion immer unterhalb der Umlenkstelle liegen, wobei das Kondensatniveau in der Verbindungsleitung, beim Arbeiten des Druckbehälters mit Überdruck, nicht unterhalb des unteren, freien Endes der Verbindungsleitung absinken bzw., beim Arbeiten des Druckbehälters mit Unterdruck, nicht über die Umlenkstelle ansteigen darf, um diese Dichtfunktion zu erfüllen. Die Druckleitung ist somit nicht durch einen Kondensatpfropf verschließbar. Demgemäß ist auch die bekannte Vorrichtung nur bei Drücken in der Nähe des Atmosphärendrucks einsetzbar.

Dampfsterilisatoren, wie sie beispielsweise in der Medizintechnik üblich sind, arbeiten mit Dampftemperaturen von bis zu 150° C (normalerweise zwischen 121° C und 134° C, was absoluten Drücken zwischen 2 und 3 bar entspricht) und mit abwechselnden Druckerhöhungen bzw. Evakuierungen (auf etwa 60 mbar, absolut) der Sterlisierkammer, um sicherzustellen, daß der heiße Dampf auch in jede Pore des zu sterlisierenden Gegenstandes eindringt. Beim Betrieb derartiger Dampfsterilisatoren ist es zweckmäßig, nicht nur die erforderliche Dampftemperatur sondern auch den Druck während der Beriebszyklen zu überwachen. Bei den verwendeten hohen Temperaturen sind jedoch die normalerweise eingesetzten, relativ preisgünstigen Druckfühler nicht geeignet bzw. nicht betriebssicher. Es müßten somit die temperaturbeständigen, jedoch teureren Druckfühler eingesetzt werden, die die Dampfsterilisatoren zusätzlich verteuern würden.

Der Erfindung liegt somit die Aufgabe zugrunde, eine relativ preisgünstige Druckmeßeinrichtung bereitzustellen, die in der Lage ist, höhere Betriebstemperaturen abzuschirmen, und die für höhere Drücke bzw. Druckschwankungen einsetzbar ist, wie sie beispielsweise bei Dampfsterilisatoren auftreten.

Die Aufgabe wird durch die kennzeichnenden Merkmale des Patentanspruchs 1 gelöst.

Durch die erfindungsgemäß verwendete Konensatabscheideund Rückhaltevorrichtung, die in eine vom Dampfsterilisator zum Druckfühler führende Druckleitung eingeschaltet ist, wird während des ersten Betriebszyklus ein Kondensatpfropf abgeschieden und während der Evakuierung zwischen Druckfühler und Sterilisierkammer zurückgehalten. Dieser Kondensatpfropf wirkt als Barriere gegen einen direkten Kontakt des einschießenden heißen Dampfes mit dem Druckfühler während der Druckerhöhung. Es konnte überraschenderweise festgstellt werden, daß dieser Kondensatpfropf in der Lage ist, den Druckfühler zumindest so weit gegen die Temperatur des Heißdampfes zu isolieren, daß einer der relativ preisgünstigen, jedoch temperaturempfindlichen Druckfühler verwendet werden kann.

Aus der DE-A 21 08 089 ist zwar bereits eine Einrichtung zum Messen des Druckes von warmer dampfhaltiger Luft bekannt, bei der ein gegen Dampf nicht widerstandsfähiges Membranmanometer verwendet wird. Um die empfindliche Membran dieses Manometers gegen den Dampfgehalt der Luft zu schützen, wird ein Labyrinthtopf in die Verbindungsleitung zum Manometer eingeschaltet. Einlaßleitung und Auslaßleitung münden frei in den oberen Bereich des Labyrinthtopfes, während sich im unteren Bereich ein Siphon befindet, der zum Ableiten des ausfallenden Kondensats dient. Es ist ein Überlauf vorgesehen, der verhindert, daß sich der Kondensatspiegel im Labyrinthtopf so weit erhöht, daß die Verbindungsleitung durch eine Kondensatmenge verschlossen wird. Der Dampf aus dem zu messenden Raum liegt somit voll am Manometer an, soll jedoch durch die Anordnung des Labyrinthtopfes soweit gekühlt werden, daß er keinen Schaden mehr anrichten kann. Die bekannte Einrichtung eignet sich jedoch nur für Drücke im Bereich von 1 bar. Bei Verwendung in Überdruck-, Unterdruck- und Gleichdruckbehältern und bei Verwendung insbesondere für Dampfsterilisatoren reicht die Abkühlungswirkung des Labyrinthtopfes für den bei höherem Druck notwendigerweise auch heißeren Dampf nicht aus, um die Membran zuverlässig zu schützen.

Auch die US-A-1 640 600 zeigt eine Druckmeßeinrichtung, die nicht für Behälter einsetzbar ist, bei denen Unterdruck auftritt. Die bekannte Druckmeßeinrichtung weist einen Labrinthtopf auf, der in verschiedene Kammern derart aufgeteilt ist, daß sich zwischen dem Druckraum und dem Druckfühler eine Wassersäule befindet. Die Wassersäule besteht aus eingefülltem Wassser, das bis zur Oberkante einer zum Druckraum abfallenden Falleitung steht. Wird evakuiert, so wird der gesamte Wasserpfropf ausgesaugt.

Durch das Umgehungsrohr gemäß Anspruch 2 wird einerseits der Meßfehler ausgeglichen, der durch das Gewicht der Kondensatsäule ausgelöst wird. Zum anderen wird durch dieses Umgehungsrohr die Rückhaltung des Kondensats beim Evakuieren verbessert, da dadurch auf beiden Seiten des Kondensatpropfes evakuiert werden kann. Durch den geringen Durchmesser des Umgehungsrohres wird einerseits die Durchflußmenge stark begrenzt, andererseits wird der Dampf abgekühlt, so daß im wesentlichen nur nicht kondensierbare, d.h. ideale, Gase am Druckfühler anliegen, die eine geringe Wärmekapazität aufweisen. Dadurch wird eine unzulässig hohe Wärmebelastung des Druckfühlers, die zur Fehlmessung oder sogar zur Beschädigung des Druckfühlers führen kann, verhindert.

Das in Anspruch 3 beschriebene Abscheidegefäß stellt eine besonders einfache und kostengünstige Möglichkeit zur Kondensatabscheidung dar.

Durch die Ausgestaltung nach Anspruch 4 wird die Wirkung der Abscheide- und Rückhalteeinrichtung im Hinblick auf die Rückhaltung des Kondensats wesentlich verbessert. Durch diese Ausgestaltung wird sichergestellt, daß abgeschiedenes Kondensat immer in den zum Druckfühler führenden Abschnitt der Druckleitung unter dem Einfluß der Schwerkraft fließen kann, wobei sich zweckmäßigerweise an diesen von unten einmündenden Abschnitt, gemäß Anspruch 5, die Umlenkstelle anschließt.

In der Ausgestaltung nach Anspruch 6 wird die Abscheidewirkung wesentlich verbessert, da der in das Abscheidegefäß einströmende heiße Dampf zunächst gegen eine Wandung des Abscheidegefäßes prallt, wodurch eine effektive Abkühlung eingeleitet wird. Darüberhinaus wird durch diese Ausgestaltung sichergestellt, daß der Dampf nicht in den zum Druckfühler führenden Abschnitt der Druckleitung eingeblasen werden kann.

Der Anspruch 7 beschreibt eine besonders bevorzugte, konstruktiv einfache Ausgestaltung, bei der die Druckleitung bereits durch relativ geringe Mengen Kondensat absperrbar ist.

Die Ausgestaltung nach Anspruch 8 isoliert den Druckfühler auch gegen eine Berührung mit dem heißen Kondensat, da durch die Anordnung des Druckleitungsabschnittes oberhalb des Kondensatnivaus ein Polster aus kalten Gasen verbleibt, das durch die Kondensatsäule zwar komprimiert, jedoch nicht beseitigt wird.

Die Anordnung des Umgehungsrohres gemäß Anspuch 9 hat sich als besonders zweckmäßig erwiesen.

Wenn gewünscht, können, wie in Anspruch 10 angegeben, die Abschnitte der Druckleitung aktiv oder passiv gekühlt werden.

Die Ausgestaltung nach Anspruch 11 unterstützt das Aufrechterhalten eines vorbestimmten Kondensatnivaus.

Ein Ausführungsbeispiel der Erfindung wird nachfolgend anhand einer einzigen, schematischen Zeichnung näher erläutert.

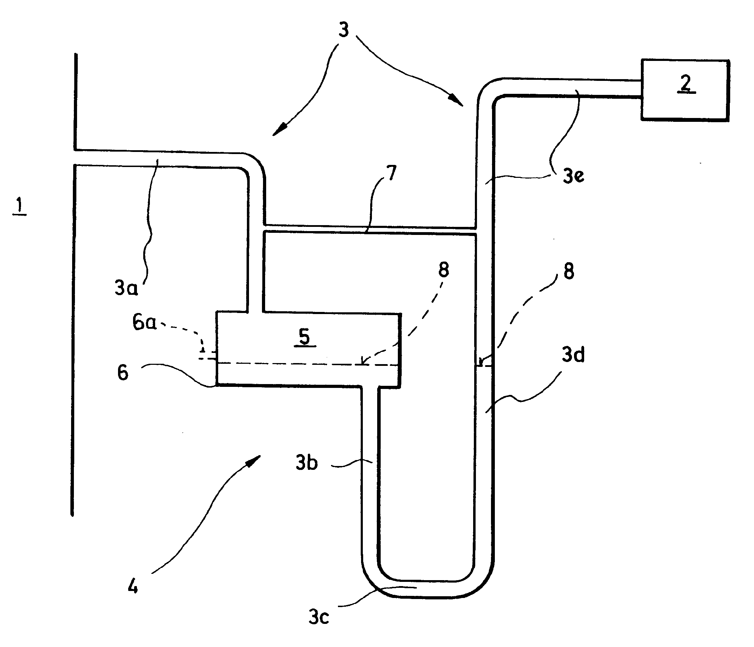

In der Zeichnung ist ein als Druckbehälter ausgestalteter Dampfbekälter 1, insbesondere eine Sterilisierkammer eines Dampfsterilisators ersichtlich, von dem jedoch lediglich eine Wand schematisch dargestellt ist. Das Innere des Dampfbehälters 1 bzw. der Sterilisierkammer ist mit einem der handelsüblichen, relativ temperaturempfindlichen Druckfühler 2 über eine Druckleitung 3 druckübertragend verbunden. In die Druckleitung 3 ist eine Abscheide- und Rückhalteeinrichtung 4 für das Kondensat eingeschaltet. Die Abscheide- und Rückhalteeinrichtung 4 enthält ein Abscheidegefäß 5. Das Abscheidegefäß 5 weist eine im wesentlichen zyylindrische Wandung 6 auf und ist liegend angeordnet. Die Wandung 6 besteht aus einem wärmeleitenden Material, bevorzugt Metall. Nach Bedarf kann im Abscheidegefäß 5 auch ein gestrichelt gezeichneter Überlauf 6a für überschüssiges Kondensat vorgesehen sein, der entweder absperrbar oder, z.B. durch eine Verbindung mit der Sterilisierkammer, druckbeaufschlagt ist.

In das Abscheidegefäß 5 mündet von oben, d.h. mit einem Fallrohrstück, ein erster Druckleitungsabschnitt 3a, der direkt mit dem Dampfbehälter 1 in Verbindung steht. Der erste Druckleitungsabschnitt 3a mündet mit einem fallenden Abschnitt in das Abscheidegefäß 5, kann jedoch auch einen Krümmer enthalten.

Die Druckleitung 3 verläßt das Abscheidegefäß 5 mit einem von unten her in das Abscheidegefäß 5 einmündenden, zweiten Druckleitungsabschnitt 3b. Die Mündungsstellen des ersten Druckleitungsabschnittes 3a und des zweiten Druckleitungsabschnittes 3b sind außer im vertikalen Mündungsniveau auch seitlich versetzt, so daß sowohl der Einmündung des ersten Druckleitungsabschnittes 3a als auch der Mündung des zweiten Druckleitungsabschnittes 3b jeweils ein Bereich der Wandung des Abscheidegefäßes 5 gegenüberliegt.

Der zweite Druckleitungsabschnitt 3b ist als Fallrohr ausgebildet und mit einem Krümmer 3c verbunden. Der Krümmer 3c ist als U-förmig gebogener Druckleitungsabschnitt ausgebildet, wobei der Krümmungsradius des Krümmers möglichst klein ist, um zu erreichen, daß der Krümmer 3c bereits durch geringe Mengen Kondensat verschlossen werden kann. An den Krümmer 3c schließt sich ein als Steigleitung ausgebildeter, dritter Druckleitungsabschnitt 3d an, der nach oben zumindest bis zum Abscheidegefäß 5 reicht. Oberhalb des Abscheidegefäßes 5 schließt sich an den dritten Druckleitungsabschnitt 3d ein vierter Druckleitungsabschnitt 3e an, der direkt zum Druckfühler 2 führt. Der vierte Druckleitungsabschnitt 3e kann in seinem oberen Bereich waagerecht geführt werden; er kann jedoch auch senkrecht verlaufen oder andere Lagen einnehmen, wenn dies erforderlich ist.

Oberhalb des Abscheidegefäßes 5 ist der fallende Bereich des ersten Druckleitungsabschnittes 3a mit dem vierten Druckleitungsabschnitt 3e durch ein Umgehungsrohr 7 verbunden. Das Umgehungsrohr 7 weist einen gegenüber dem Querschnitt der Druckleitung 3 derart verringerten Querschnitt auf, daß der Durchtritt von Dampf weitgehend verhindert, jedoch ein Druckausgleich möglich ist. Darüber hinaus darf der Querschnitt des Umgehungsrohres 7 jedoch nicht so klein sein, daß sich das Umgehungsrohr 7 zusetzen kann. Für Druckleitungen mit einem Innendurchmesser von etwa 3 bis 6 mm, bevorzugt 4 mm, sollte die Umgehungsleitung 7 einen Innendurchmesser von 1 bis 2 mm, bevorzugt 1,2 mm aufweisen.

Die Länge des vierten Druckleitungsabschnittes 3e, gegebenenfalls zusammen mit derjenigen Länge des dritten Druckleitungsabschnittes 3d, die sich bei Druckausgleich im wesentlichen über dem Kondensatspiegel befindet, sollte derart auf den Durchmesser der Druckleitung abgestimmt sein, daß während der Druckerhöhung im Dampfbehälter zwischen dem Kondensat und dem Druckfühler 2 ein Gaspolster verbleibt. Das Gaspolster enthält zum einen Luft, die während der Betriebszyklen über die Umgehungsleitung 7 in die Druckleitungsabschnitte 3d bzw. 3e gelangen kann, und zum anderen Gase, die im Dampf enthalten sind und bei der Kondensation zurückbleiben.

Beim Betrieb des Dampfsterilisators wird heißer, unter Druck stehender Dampf in die Sterilisierkammer 1 eingeleitet und gelangt über den ersten Druckleitungsabschnitt 3a in das Abscheidegefäß 5. Dort kühlt sich der Dampf ab, so daß bereits eine bestimmte Menge Kondensat ausfällt, das aus dem Abscheidegefäß 5 in den zweiten Druckleitungsabschnitt 3b fließt und sich im Krümmer 3c sammelt. Durch den geringen Krümmungsradius des Krümmers 3c genügt eine relativ geringe Menge Kondensat, um den Kondensatspiegel soweit ansteigen zu lassen, daß er den Durchmesser der Druckleitung 3 im Bereich des Krümmers 3c übersteigt. Damit wird ein Kondensatpfropf geschaffen, der den einströmenden heißen Dampf davon abhält, in den dritten und vierten Druckleitungsabschnitt 3d bzw. 3e einzuströmen. Der Kondensatpfropf wird lediglich in Abhängigkeit vom Druck in der Druckkammer 1 in Richtung auf den Druckfühler 2 verschoben, so daß der herrschende Druck auf den Druckfühler 2 übertragen wird. Gleichzeitig findet ein Druckausgleich zwischen beiden Seiten des Kondensatpfropfes über die Umgehungsleitung 7 statt. Damit wird erreicht, daß sich beidseitig des Kondensatpfropfes im wesentlichen der gleiche Druck aufbaut und somit derjenige Meßfehler vermieden wird, der zu einem um das Gewicht der stehenden Kondensatsäule verringerten Anzeigewert des Druckes am Druckfühler führen würde.

Während des darauffolgenden Evakuierens wird für den Fall, daß der Druckausgleich über das Umgehungsrohr 7 nicht schnell genug erfolgen kann, das im Krümmer 3c bzw. im Rohrabschnitt 3d befindliche Kondensat durch Expansion des Gaspolsters im Abschnitt 3e in den Abscheidebehälter 5 verdrängt und dort durch Schwerkraftabscheidung vom Gas getrennt. Das Gas kann über den Druckleitungsabschnitt 3a zur Kammer hin entweichen.

Infolge der Druckabsenkung im System während des Evakuierungsvorganges verdampft ein Teil des Kondensates im Abscheidegefäß 5. Wegen zunehmender Auskühlung des Kondensates und des Abscheidegefäßes 5 kommt jedoch der Abkochungsvorgang zum Stillstand und es bleibt eine gewisse Menge Kondensat, beispielsweise in Höhe des gezeichneten Kondensatniveaus 8, im System zurück, das beim darauffolgenden Erhöhen des Druckes bereits von Anfang an für eine wirksame Isolierung des Druckfühlers 2 vom auftreffenden heißen Dampf sorgt. Darüber hinaus wird sich eine weitere Menge Kondensat abscheiden, die jedoch beim darauffolgenden Evakuieren vollständig oder zum Teil wieder verdampft.

Bei zweckmäßiger Ausgestaltung der Abmessungen des Abscheidegefäßes 5 und bei der Wahl eines geeigneten Werkstoffes mit ausreichender Wärmeleitfähigkeit wird sich die Kondensatmenge im System nicht so weit verändern, daß die Funktionsweise gefährdet wäre. Zur Sicherheit kann jedoch der Druckfühler über dem Niveau der Einmündung des ersten Druckleitungsabschnittes 3a in den Dampfbehälter 1, bzw. über den höchsten Punkt des ersten Druckleitungsabschnittes 3a, angeordnet oder durch den Überlauf 6a ein maximales Kondensatniveau festgelegt werden. Dabei sollte sich zumindest der vierte Druckleitungsabschnitt 3e vollständig oberhalb des maximalen Kondensatniveaus befinden. Auch das Umgehungsrohr 7 sollte oberhalb dieses maximalen Kondensatniveaus angeordnet sein.

Zum Verbessern der Kondensatabscheidung kann die Druckleitung in allen ihren Abschnitten, oder nur ausgewählte Druckleitungsabschnitte aktiv, z.B. durch einen Kühlmantel, gekühlt werden oder zum passiven Kühlen beispielsweise als Rippenrohre oder dergleichen mit vergrößerter Wärmetauschfläche ausgebildet sein. Auch das Abscheide- und Rückhaltegefäß 4 kann aktiv oder passiv gekühlt werden.

Bei sorgfältiger Bemessung des gesamten Systems und bei Inkaufnahme des Meßfehlers ist das Umgehungsrohr 7 unter Umständen entbehrlich. Auch das Abscheidegefäß 5 kann beispielsweise mit Einbauten versehen werden oder eine abweichende Form aufweisen. Andere bekannte Kondensatabscheidungs- und Rückhalteeinrichtungen sind verwendbar. Die Umlenkstelle kann darüber hinaus beispielsweise auch eine V-Form aufweisen oder als 180°-Biegung ausgebildet sein. Schließlich kann das Umgehungsrohr 7 vom oberen Bereich des Abscheidegefäßes oder von einer anderen Stelle in den dritten bzw. auch den vierten Druckleitungsabschnitt führen. Die Anzahl und Anordnung der Druckleitungsabschnitte kann weiterhin entsprechend konstruktiven Besonderheiten variiert werden. Die anmeldungsgemäße Druckmeßeinrichtung ist ferner für alle Überdruck-, Unterdruck- und Gleichdruckbehälter einsetzbar, bei denen die anhand einer Sterilisierkammer erläuterten Probleme auftreten.

**Patentansprüche**

1. Druckmeßeinrichtung, mit einem Druckfühler (2) zum Messen des Drucks in einem Druckbehälter (1), der kondensierbare Gase oder eine Mischung aus kondensierbaren Gasen und nicht kondensierbaren Gasen enthält, und der zwischen Überdruck, Unterdruck und Gleichdruck wechselnden Drücken ausgesetzt ist, wobei der Druckfühler (2) über eine eine im wesentlichen vertikal ausgerichtete Umlenkstelle (3c) enthaltende Druckleitung (3) mit dem Druckbehälter (1) verbunden ist, und wobei eine Rückhalteeinrichtung zum Rückhalten einer Kondensatmenge unter Ausbildung eines Kondensatniveaus (8) vorgesehen ist, **dadurch gekennzeichnet,** daß, insbesondere bei Ausbildung des Druckbehälters als Sterilisierkammer eines Dampfsterilisators, die Rückhalteeinrichtung als eine Abscheide- und Rückhalteeinrichtung (4) ausgebildet ist und in die Druckleitung (3) eingeschaltet ist, und die Umlenkstelle (3c) der Druckleitung (3) unterhalb des Kondensatniveaus (8) zum Rückhalten der die Druckleitung (3) zwischen dem Druckbehälter (1) und dem Druckfühler (2) verschließenden Kondensatmenge angeordnet ist.

2. Druckmeßeinrichtung nach Anspruch 1, **dadurch gekennzeichnet,** daß zwischen dem Druckbehälter (1) und dem Druckfühler (2) eine Umgehungsleitung (7) zum Umgehen der Abscheide- und Rückhalteeinrichtung (4) oberhalb des Kondensatniveaus (8) vorgesehen ist, deren Querschnitt kleiner als der Querschnitt der Druckleitung (3) ist.

3. Druckmeßeinrichtung nach einem der Ansprüche 1 oder 2, **dadurch gekennzeichnet,** daß die Abscheide- und Rückhalteeinrichtung (4) ein Abscheidegefäß (5) enthält.

4. Druckmeßeinrichtung nach Anspruch 3, **dadurch gekennzeichnet,** daß in das Abscheidegefäß (5) von oben ein erster Druckleitungsabschnitt (3a) vom Druckbehälter (1) und von untein ein zweiter Druckleitungsabschnitt (3b) vom Druckfühler (2) mündet.

5. Druckmeßeinrichtnug nach Anspruch 4, **dadurch gekennzeichnet,** daß der zweite Druckleitungsabschnitt (3b) zwischen dem Abscheidegefäß (5) und der Umlenkstelle (3c) angeordnet ist.

6. Druckmeßeinrichtung nach Anspruch 4 oder 5, **dadurch gekennzeichnet,** daß die Einmündungen des ersten und zweiten Druckleitungsabschnittes (3a, 3b) in das Abscheidegefäß (5) seitlich zueinander versetzt sind.

7. Druckmeßeinrichtung nach einem der Ansprüche 4 bis 6, **dadurch gekennzeichnet,** daß die Umlenkstelle als U-förmiger Krümmer (3c) mit einem kleinen Krümmungsradius ausgebildet ist, der zwischen dem zweiten Druckleitungsbschnitt (3b) und einem nach oben in Richtung auf den Druckfühler (2) führenden dritten Druckleitungsabschnitt (3d) angeordnet ist.

8. Druckmeßeinrichtung nach einem der Ansprüche 1 bis 7, **dadurch gekennzeichnet,** daß ein vierter Druckleitungsabschnitt (3e) vorgesehen ist, der oberhalb des Kondensatniveaus (8) mit dem zweiten Druckleitungsabschnitt (3b) verbunden ist und zum Druckfühler (2) führt.

9. Druckmeßeinrichtung nach einem der Ansprüche 2 bis 8, **dadurch gekennzeichnet,** daß die Umgehungsleitung (7) von einem nach nuten führenden Bereich des ersten Druckleitungsabschnittes (3a) zum vierten Druckleitungsabschnitt (3e) führt.

10. Druckmeßeinrichtung nach einem der Ansprüche 1 bis 9, **dadurch gekennzeichnet,** daß mindestens ein Druckleitungsabschnitt (3a bis e) aktiv oder passiv kühlbar ist.

11. Druckmeßeinrichtnug nach einem der Ansprüche 1 bis 10, dadurch gekennzeichnet, daß der Abscheide- und Rückhalteeinrichtnug (4) oberhalb eines vorbestimmten Kondensatniveaus (8) ein bevorzugt im Abscheidegefäß (5) angeordneter Überlauf (6a) für überschüssiges Kondensat zugeordnet ist.

**Claims**

1. Pressure gauge, with a pressure sensor (2) for measuring the pressure in a pressure vessel (1), which contains condensable gases or a mixture of condensable and non-condensable gases, and which is subjected to pressures varying between excess pressure, partial vacuum and atmospheric pressure, the pressure sensor (2) being connected to the pressure vessel (1) via a pressure line (3) containing an essentially vertically oriented loop area (3c), and a retention arrangement being provided for retention of a condensate quantity in order to form a condensate level (8), characterised in that, especially when the pressure vessel is designed as the sterilising chamber of a steam steriliser, the retention arrangement is designed as a separating and retention arrangement (4) and is connected into the pressure line (3), and the loop area (3c) of the pressure line (3) is arranged below the condensate level (8) for retaining the condensate quantity closing the pressure line (3) between the pressure vessel (1) and the pressure sensor (2).

2. Pressure gauge according to Claim 1, characterised in that, between the pressure vessel (1) and the pressure sensor (2), a bypass line (7) is provided for bypassing the separating and retention arrangement (4) above the condensate level (8), the cross-section of the bypass line being smaller than the cross-section of the pressure line (3).

3. Pressure gauge according to either of Claims 1 and 2, characterised in that the separating and retention arrangement (4) contains a separating vessel (5).

4. Pressure gauge according to Claim 3, characterised in that a first pressure line section (3a) from the pressure vessel (1) opens into the separating vessel (5) from above and a second pressure line section (3b) from the pressure sensor (2) opens into the separating vessel (5) from below.

5. Pressure gauge according to Claim 4, characterised in that the second pressure line section (3b) is arranged between the separating vessel (5) and the loop area (3c).

6. Pressure gauge according to Claim 4 or 5, characterised in that the openings of the first and second pressure line sections (3a, 3b) into the separating vessel (5) are laterally offset relative to one another.

7. Pressure gauge according to one of Claims 4 to 6, characterised in that the loop area is designed as a U-shaped bend (3c) with a small radius of curvature, which is arranged between the second pressure line section (3b) and a third pressure line section (3d) leading upwards in the direction of the pressure sensor (2).

8. Pressure gauge according to one of Claims 1 to 7, characterised in that a fourth pressure line section (3e) is provided, which is connected above the condensate level (8) to the second pressure line section (3b) and leads to the pressure sensor (2).

9. Pressure gauge according to one of Claims 2 to 8, characterised in that the bypass line (7) leads from a downwardly extending area of the first pressure line section (3a) to the fourth pressure line section (3e).

10. Pressure gauge according to one of Claims 1 to 9, characterised in that at least one pressure line section (3a to e) can be actively or passively cooled.

11. Pressure gauge according to one of Claims 1 to 10, characterised in that the separating and retention arrangement (4) is allocated, above a predetermined condensate level (8), an overflow (6a) for excess condensate, which overflow (6a) is preferably arranged in the separating vessel (5).

**Revendications**

1. Dispositif pour mesurer une pression comprenant un palpeur (2) pour mesurer la pression à l'intérieur d'un réservoir sous pression (1) qui contient des gaz pouvant être condensés ou un mélange constitué de gaz pouvant être condensés et de gaz ne pouvant pas être condensés et qui est exposé à des pressions qui alternent entre la surpression, le vide partiel et la pression d'équilibre, ledit palpeur (2) étant relié au réservoir (1) par une conduite (3) sous pression contenant un point de dérivation (3c) orienté en direction sensiblement verticale, un dispositif de retenue pour la rétention d'une certaine quantité de condensat avec formation d'un niveau (8) ayant été prévu, caractérisé par le fait que, en particulier si le réservoir sous pression est conformé en chambre de stérilisation d'un stérilisateur à vapeur, le dispositif de retenue est réalisé sous la forme d'un dispositif de séparation et de rétention (4) et monté dans la conduite sous pression (3), et que le point de dérivation (3c) de la conduite sous pression est disposé sous le niveau (8) du condensat afin de retenir la quantité de condensat qui ferme la conduite (3) entre le réservoir (1) et le palpeur (2).

2. Dispositif pour mesurer une pression selon la revendication 1, caractérisé par le fait qu'entre le réservoir (1) et le palpeur (2) est prévue, au-dessus du niveau (8) du condensat, une conduite de dérivation (7) destinée à contourner le dispositif de séparation et de retenue (4), la section transversale de cette conduite (7) étant inférieure à la section transversale de la conduite de pression (3).

3. Dispositif pour mesurer une pression selon l'une des revendications 1 ou 2, caractérisé par le fait que le dispositif (4) de séparation et de retenue contient un vase de séparation (5).

4. Dispositif pour mesurer une pression selon la revendication 3, caractérisé par le fait que, dans le vase de séparation (5) débouchent par le haut, une première section (3a) de la conduite sous pression venant du réservoir (1) et par le bas une deuxième section (3b) de la conduite sous pression venant du palpeur (2).

5. Dispositif pour mesurer une pression selon la revendication 4, caractérisé par le fait que la deuxième section (3b) de la conduite sous pression est disposée entre le vase (5) et le point de dérivation (3c).

6. Dispositif pour mesurer une pression selon la revendication 4 ou 5, caractérisé par le fait que les entrées dans le vase de séparation (5) de la première et de la deuxième sections (3a, 3b) de la conduite sous pression sont décalées latéralement l'une par rapport à l'autre.

7. Dispositif pour mesurer une pression selon l'une des revendications 4 à 6, caractérisé par le fait que le point de dérivation est réalisé sous la forme d'un coude (3c) en forme de U à faible rayon de pliage, ce coude étant disposé entre la deuxième section (3b) de la conduite sous pression et une troisième section (3d) de la même conduite sous pression conduisant vers le haut en direction du palpeur (2).

8. Dispositif pour mesurer une pression selon l'une des revendications 1 à 7, caractérisé par le fait qu'il a été prévu une quatrième section (3e) de la conduite sous pression qui est reliée, au-dessus du niveau (8) du condensat, à la deuxième section (3b) de la conduite sous pression et qui conduit vers le palpeur (2).

9. Dispositif pour mesurer une pression selon l'une des revendications 2 à 8, caractérisé par le fait que la conduite de dérivation (7) conduit d'une zone orientée vers le bas de la première section (3a) vers la quatrième section (3e) de la conduite sous pression.

10. Dispositif pour mesurer une pression selon l'une des revendications 1 à 9, caractérisé par le fait qu'au moins une section (3a à e) de la conduite sous pression peut être réfrigérée de manière active ou passive.

11. Dispositif pour mesurer une pression selon l'une des revendications 1 à 10, caractérisé par le fait que le dispositif de séparation et de rétenue (4) est associé à un trop-plein (6a) pour le condensat en excès, ce trop-plein étant de préférence disposé dans le vase (5) au-dessus du niveau (8) du condensat.